# EUROPEAN PATENT APPLICATION

(11) **EP 2 849 103 A2**
(43) Date of publication of application: **18.03.2015**
(21) Application number: 14183550.4
(22) Date of filing: 04.09.2014
(51) Int. Cl.: G06F 19/00, G06F 17/20

(54) **An arrangement and a method for creating a synthesis from numerical data and textual information**

(30) Priority: 13.09.2013 GB 201316286
(71) Applicant: Vivago Oy, 02600 Espoo (FI)
(72) Inventor: Cederström, Eelis, 00400 Helsinki (FI); Oittinen, Sami, 02760 Espoo (FI); Kylmä, Anna Kaisa, 00380 Helsinki (FI)
(74) Representative: IPR Partners Ltd

(57) **Abstract**

The invention relates generally to an arrangement and a method for creating a synthesis from numerical data and textual information, and more particularly, the invention relates to an arrangement and a method for creating a synthesis from numerical data and textual information relating to wellbeing of an individual. The arrangement according to the present invention comprises a first information in a numerical form provided by a wellbeing device, for example, a second information comprising free-form text in natural language format provided by caregiving personnel, for example, and a control entity for obtaining the first information and the second information, wherein the control entity is arranged to semantically analyze said free-form text in natural language format of said second information in order to create a synthesis from said first information and said second information.

## Description

### Technical field

Generally, the invention relates to an arrangement and a method for creating a synthesis from numerical data and textual information, and more particularly, the invention relates to an arrangement and a method for creating a synthesis from numerical data and textual information relating to wellbeing of an individual.

### Background technology

In recent century, life expectancy for humans in developed nations has rapidly increased and people are also staying healthy to a more advanced age. Though, many elderly people want to live in their own home as long as possible, aging itself brings changes to a health and this is why several elderly people may fall into home health care services.

Various types of home care services are municipally and commercially provided, such as custodial care helping with daily living activities comprising meal, bathing, laundry, cleaning, shopping, and safety service; health care services giving medical care, such as medication management, wound care, administration of injections and IVs, blood draws, ventilation and respiratory care and fall prevention assessments, just to name a few tasks. For home care service personnel, it is essential task to notice signs in a client that may tell about weakened health condition, injuries, outbreak or worsening of diseases, such as diabetes, MS or dementia.

Unfortunately, due to the lack of municipal resources, especially, home care service personnel can have a vast number of clients and, normally, each caregiver has way too little time to be familiarized with each client's situation and health care history. During a normal day at work, one caregiver may meet several new clients for the first time, and while having a tight schedule, may have only few minutes to get relevant information concerning each client, such as read his/hers medical history or other caregivers comments. The client itself may even mislead the caregiver by leaving out important facts relating to his/hers wellbeing or by belittling his/hers symptoms. In case of a memory disorder, the client may not even be able to tell relevant issues to the caregiver. Obviously, lack of knowledge or delayed response to alarming signs in client's condition may lead the client, who lives at home, to a serious situation or it may delay beginning of disease treatment.

### Summary of the invention

It is an object of the present invention to implement such a solution, that previously mentioned drawbacks of the prior art could be diminished. In particular, the invention is implied to solve how up-to-date and relevant information from various sources can be gathered and provide to a caregiver in an easily understandable form.

The objective of the invention is met by the features disclosed in the independent patent claims.

An arrangement for creating a synthesis from numerical data and textual information relating to wellbeing of an individual according to the present invention is characterized by the features of claim 1.

According to an embodiment of the invention, an arrangement for creating a synthesis from numerical data and textual information relating to wellbeing of an individual comprises a first information in a numerical form, a second information in a textual form, and an control entity for obtaining said first information and said second information, wherein said control entity is arrange to semantically analyze said free-form text in natural language format of said second information in order to create a synthesis from said first information and said second information.

In an embodiment, the first information is provided by a wellbeing device, which is arranged to monitor said individual's wellbeing. This feature is advantageous, because typically, wellbeing devices, which are arranged to monitor several things, such as the user's activity and circadian rhythm, can be worn long periods of time, which provides a lot of data. In addition, the health condition of the user and, especially, changes in the condition may be noticed by means of the wellbeing devices, which makes them valuable aid in everyday health monitoring. Most of the wellbeing devices comprise an alarm function, which can alarm or provide means to the user to alarm help in case of an emergency, such as falling.

In another embodiment, the second information is provided by caregiver personnel. Any kind of caregiver providing help to the client typically writes a summary for other caregivers and/or her/himself about the visit comprising e.g. what has been done and comments about the client's condition, especially in situation, where the client is an elderly person living home alone and/or having a trauma, disability and/or disease. Respectively, caregivers coming to visit the client would preferably read comments written by other caregivers in order to gain relevant information about the client's overall situation.

In another embodiment, the arrangement according to the present invention further comprises one or more external measurement devices arranged to provide a third information. In case of an elder people, various kinds of measurement devices may be used daily and/or weekly providing information about the client's health, such as a scale, a body composition measurement device, a heart rate monitor, a pedometer, a blood measurement device, a blood glucose meter, sleep tracking means, a spirometer, an alcometer, a breathalyzer, EEG (Electroencephalography) and ECG (Electrocardiography).

Yet, in another embodiment, the arrangement according to the present invention further comprises one or more external databases arranged to provide a fourth information. This feature is advantageous, because typically various authorities and private operator can have interesting information relating to the client, which can be included in the obtained information in order to create a synthesis. As well, the other data obtained and/or processed by the arrangement according to the present invention can be further transferred to one or more external databases.

Yet, in another embodiment, the synthesis provided by the arrangement according to the present invention is provided in a textual form.

A method for creating a synthesis from numerical data and textual information relating to wellbeing of an individual according to the present invention is characterized by the features of claim 8.

According to an embodiment of the invention, a method for creating a synthesis from numerical data and textual information relating to wellbeing of an individual comprises at least following steps:
- obtaining information comprising data in a numerical form and free-form text in natural language format,
- converting said data in a numerical form into a textual form,
- semantically analyzing and/or formalizing said free-form text in natural language format,
- creating a synthesis from said data in a numerical form and said semantically analyzed and/or formalized free-form text in natural language format,
- providing said synthesis to a user.

In another embodiment, the method according to the present invention comprises a further step of selecting a time period for obtained information. This feature is advantageous, because it enables creation of a synthesis for different purposes. For example, a nurse may benefit of a synthesis from the past two weeks, whereas the synthesis from past year may provide a wide insight into the overall situation of the client.

Some preferable embodiments of the invention are described in the dependent claims.

Significant advantages can be achieved with the present invention when compared to the prior art solution. The arrangement and the method for creating a synthesis may provide for caregivers an easy and effective way to gather and produce relevant information relating to a client's condition, changes in health, circadian rhythm and activity. The invention can be arranged to gather information from different sources and to form it into a one entity, which may be clear and easily readable.

In addition, the invention may efficiently perform health condition monitoring of the client and it may help caregiver to notice alarming signs in client's health and/or condition and, in that way, to prevent the emergence of serious situations and/or accidents.

Furthermore, the present invention may be efficient for detecting and bringing forth weak signals, i.e. first signs of forthcoming chance relating to a client's wellbeing and/or health. Both short-term and long-term trends in input topics and optionally in related responsive actions may also be inspected.

In the present document, the term "client", hereafter also referred as "individual", refers to a person typically living at home or a homelike environment, whose wellbeing is monitored by using one or more appropriate monitoring means, such as a wellbeing device, and who has any kind of professional and/or unprofessional home care service relating to his/her wellbeing and/or health.

In the present document, the term "wellbeing" may refer to several monitored vital signs or other monitorable variable/variables, such as, but not limited to, activity, sleep detection and circadian rhythm, relating to wellbeing of an individual or indicating chances in individual's health.

Further, in the present document, the term "caregiver" refers to at least one person of home care service personnel, which can be professional, such as, but not limited to, nurses, home care aides, social workers, physical therapists, occupational therapists, speech and language pathologists and dietitians, or an unprofessional person aiding with daily living activities or for keeping company, for example.

The expression "numerical form" refers herein to any kind of value, which can be measured and/or compared to other values, as numbers, decimal numbers, integers, binary numbers, rational numbers, and irrational numbers.

The expression "electronic text" refers herein various electronic formats comprising text, such as a Word file, text file, Portable Document Format file or another suitable text file format.

The expression "textual form" refers herein, in a more general sense, words in a meaningful order to convey information. The textual form can comprise electronic text as described above and/or text in a paper.

The expression "a number of" refers herein to any positive integer starting from one (1), e.g. to one, two, or three.

The terms "a" and "an", as used herein, are defined as one or more than one.

### Short description of the drawings

Next, the invention is described in more detail with reference to the appended drawings, in which
Fig. 1 illustrates the concept of an embodiment of the present invention;
Fig. 2 illustrates internals of a control entity according to an embodiment of the present invention;
Fig. 3 is a flowchart disclosing an embodiment of a method in accordance of the present invention.

### Detailed description of the embodiments

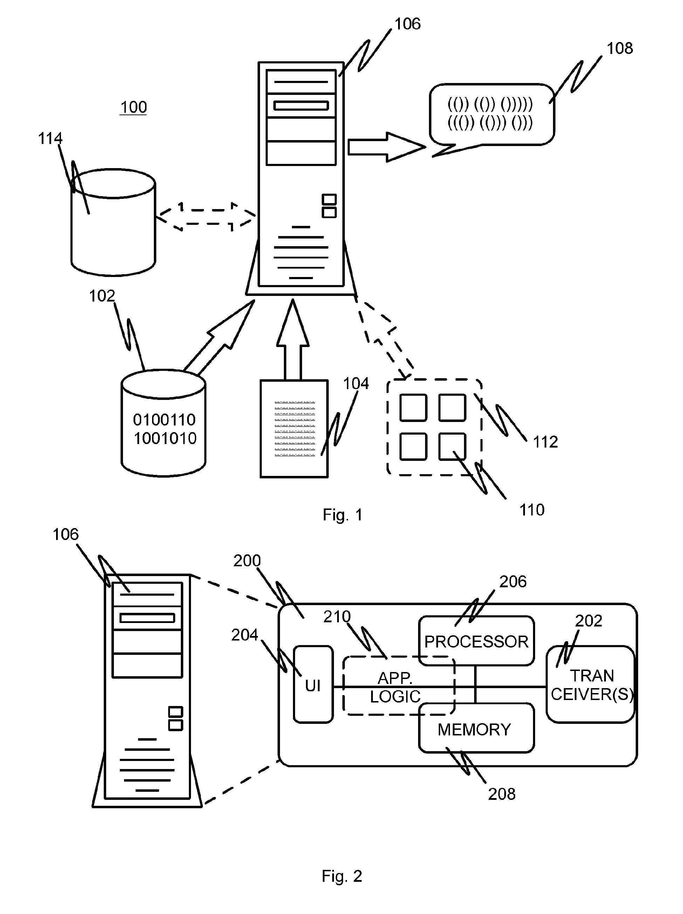

Fig. 1 illustrates the concept of an embodiment of the present invention. In one embodiment, an arrangement 100 for creating a synthesis from numerical data and textual information relating to wellbeing of an individual comprises a first information 102, a second information 104 and an control entity 106.

In a preferred embodiment, the first information 102 is in a numerical form. In another embodiment, the first information 102 can also comprise data in another form, such as a graphical form. In one embodiment, the first information relating to wellbeing of an individual is provided by a wellbeing device, which is arranged to monitor the individual's wellbeing. Such wellbeing devices may include one or more wearable devices, e.g. a wrist-held device, arranged to monitor user's wellbeing, such as activity, sleep and circadian rhythm. Typically, the circadian rhythm can be deduced based on the activity and sleep detection data. It is obvious to the person skilled in the art, that the wellbeing device can also be arranged to monitor other vital signs, such as, but not limited to, heart rate, body temperature, blood pressure and respiratory rate.

In another embodiment, the wellbeing device is also arranged to monitor other issues, such as wearing/not wearing, on/off and in range/ out of range. These data information, as well as other monitored information, can be included in wellbeing device's data related to individual's wellbeing.

Yet, in another embodiment, the wellbeing device can be arranged to monitor and transfer other information relating to the device itself, such as battery state of charge, the temperature of the initials of the device and total usage time, to name a few.

Preferably, the wellbeing device is arranged to transfer data to a base unit, such as, but not limited to, cloud, a server, a computer, a laptop, a tablet, a smartphone, another mobile device or the combination thereof. It is obvious to the person in the art that the base unit can also locate in the control entity of the present invention or the base unit can be arranged to be a part of the control entity.

In a preferred embodiment, the second information 104 comprises free-form text in natural language format and it can be an electronic text or it can be converted into an electronic text. The person skilled in the art will understand that free-form text, when provided as an electronic text, can comprise a number of text files in one or more formats comprising also metadata.

In a preferable embodiment, both the first and the second information comprise a time stamp or other time information relating to the origin of the information. In another embodiment, the creation time or time span can be traced by some other means. In another embodiment, also a third information comprises time information relating to the origin of the information.

In one embodiment, the second information is provided by caregiver personnel. The second information can be provided into the arrangement by various means, e.g. by typing it by using of a communication interface of an electronic device, such as, but not limited to, a computer, a mobile phone, a smart phone a laptop or a tablet, to name a few. In another embodiment, speech recording is used in connection with some speech recognition software, which is arranged to convert the recorded speech into an electronic text in some suitable text format. In another embodiment, hand written text and/or text written with a typewriter is scanned with optical character recognition by some suitable software. The person skilled in the art will understand that the arrangement can be arranged to obtain the second information by using different embodiments and above described embodiments can be combined in order to provide second information in an electronic text format. It is also obvious to the person skilled in the art that the second information provided as an electronic text in an electronic device can be transferred to the control entity, if the electronic device in question is not the control entity or a part of it.

In one embodiment, the arrangement further comprises one or more external measurement devices 110 arranged to provide a third information 112, which third information 112 is obtained by the control entity of the present invention and included in the obtained information for creating a synthesis. The group of external measurement devices is depicted with a dotted line in Fig. 1 to indicate the optional or additional characteristic of this embodiment.

The group of external measurement devices 110 can comprise various kinds of measurement devices, such as, but not limited to, a scale, a body composition measurement device, a heart rate monitor, a pedometer, a blood measurement device, a blood glucose meter, sleep tracking means, a spirometer, an alcometer, a breathalyzer, EEG (Electroencephalography) and ECG (Electrocardiography). The person skilled in the art will understand that various types of external device / devices can be included in or exclude from the group of external measurement devices.

The third information provided by the external measurement devises 110 can be in a numerical form, in a textual form, in a graphical form or any kind of combination thereof. Depending on a device in the group of the external devices 110, the device can comprise wireless and/ wired means for transferring data, such as Bluetooth, Wibree, ZigBee or USB. The data transfer between the external measurement devices and the control entity 106 can be arranged to be straight and/or automatic performed or the data can be first transferred via another electronic device, such as the base unit of the wellbeing device.

Yet, in another embodiment, the arrangement further comprises one or more external databases 114 arranged to provide a fourth information, which fourth information is obtained by the control entity of the present invention and included in the obtained information for creating a synthesis. The external database 114 is depicted with a dotted line in Fig. 1 to indicate the optional or additional characteristic of this embodiment.

The external database(s) can comprise data from various sources, such as, but not limited to, information provided by a wellbeing service and/or another wellbeing arrangement, health information, medical reports, anamneses, diagnoses and/or medical case summaries provided by authorities responsible of healthcare and/or private healthcare clinics and/or hospitals. The other database(s) can locate in one or more places, such as in cloud, in one or more servers and/or computer(s) and/or in another electronic device(s). The fourth data can be obtained from the external database(s) via wireless and/or wired means.

The fourth information can also comprise information relating to enterprise resource planning. It is obvious to the skilled person that the fourth information obtained from the external database(s) can be other information relating to the individual in question. The fourth data is can be in a numerical form, in a textual form, in a graphical form or any kind of combination thereof.

The fourth data is obtained from the external database(s) 114, but, as indicated with a double-headed arrow, another data, such as a synthesis, syntheses, the first information, the second information, the third information and/or the fourth information from another databases or any combination thereof, can also be transferred to the external database.

It is obvious to the skilled person that the previous described embodiments can be combined in various different ways when creating a synthesis.

In one embodiment, the first, the second, the third and/or fourth information comprises time information relating to the origin of the data. The time information is used to arranging the information in chronological order. In one embodiment, the user can select the time period for the obtained information. The feature is advantageous, because various types of synthesis can be created for different purposes. For example, a nurse may consider a synthesis based on the last two weeks period of data to be the most useful for his/her purposes. When observing the situation and development of the individual on the whole, the synthesis from a wider time period, such as six month, a year or even wider than that, may be useful.

In another embodiment, the all obtained and saved information is utilized when creating a synthesis. In one embodiment, the certain data contribution can be emphasized by giving it a higher weight. In one embodiment, more recent data gets a higher weight than older data. In another embodiment, data is weighted based on the source of the data. In an example of the present embodiment, the first information is weighted with a higher coefficient than the second information. In another example, the second information provided by a nurse gets a higher weight coefficient than the second information provided by a relative, for instance.

It is obvious to the person skilled in the art, that the above described embodiments can be combined, in other words, it is possible to provide an arrangement, which provides a synthesis creation from a selectable time period and weighing data with different coefficients based on various criteria.

The control entity 106 according to the present invention is arranged to obtain and process the first information 102, the second information 104, and, optionally or additionally, the third information 112 and/or the fourth information. The control entity 106 may be implemented with a number of computing devices, such as servers and/ or general computer devices, and it may at least partially reside in a cloud computing environment from which computational resources may be flexibly allocated upon need. It is obvious to the skilled person that the components of the control entity do not have to locate in the same place, but those components can be functionally connected with each other.

In Fig. 2 is presented, by way of example only, a block diagram of internals 200 of the control entity 106. The control entity 106 comprises data transfer means 202, such as at least one transceiver incorporating e.g. a radio part including a wireless transceiver, such as WLAN (wireless LAN), Bluetooth or a cellular like GSM (Global System for Mobile Communications)/UMTS (Universal Mobile Telecommunication System) transceiver and/or other wireless or wired data connectivity means such as one or more wired interfaces (e.g. Firewire, LAN such as Ethernet, or USB (Universal Serial Bus)) for obtaining the first, the second and, optionally, the third information, and general communications with external devices and/or a network infrastructure.

The UI (user interface) 204 may comprise data visualization element, such as a display, e.g. an (O)LED (organic LED) or LCD (liquid crystal display) display, and/or a connector to an external display, a printer or a plotter, and a data input element, such as a keyboard/ keypad/mouse/touchpad and/or other applicable control input means (e.g. a touch screen or voice control input system, or separate keys/buttons/knobs/switches) configured to provide the user of the control entity 106 with practicable data, visualization means and control, such as typing and/or editing means. The UI 204 may further include one or more loudspeakers and associated circuitry such as D/A (digital-to-analogue) converter(s) for sound output, and a microphone with A/D converter for sound input.

Furthermore, the control entity 106 comprises one or more processing devices 206 capable of processing instructions and other data, such as one or more microprocessors, microcontrollers, DSPs (digital signal processor), programmable logic chips, or a desired plurality of any of those. The processing entity 206 may thus, as a functional entity, physically comprise a plurality of mutually co-operating processors and/or a number of sub-processors connected to a central processing unit, for instance. The processing entity 206 may be configured to execute the code stored in a memory 208, which may refer to analysis software 210 in accordance with the present invention and other desired software applications.

Similarly, the memory entity 208 may be divided between one or more physical memory chips or other memory elements. The memory 208 may be configured to accommodate a computer program comprising code elements in the form of computer-executable instructions and related other data for controlling the arrangement. Further, the memory 208 may be utilized to host the first information 102, the second information 104 and, depending on embodiment, the third information 112, as well as results of the arrangement 100, i.e. the synthesis 108 and possible intermediate results. The computer program may be embodied on a carrier medium, such as a memory card or an optical disc. The memory 208 may further refer to and include other storage media, such as a preferably detachable memory card, a floppy disc, a CD-ROM, or a fixed storage medium such as a hard drive. The memory 204 may be non-volatile, e.g. ROM (read only memory), and/or volatile, e.g. RAM (random access memory), by nature.

The logic for the analysis engine and the related functionalities may indeed be implemented as software stored in the memory entity 208 and executed by the processing entity 206. The analysis entity 210 may include a number of applications, modules, and/or other software preferably having a functional interconnection. Software 210 may utilize a dedicated or a shared processor for executing the tasks thereof.

In a preferred embodiment, the natural language processing entity can be configured to semantically analyze the free-form text in natural language format by executing one or more various analyzing methods, such as, but not limited to, classification, filtering, language identification, segmentation, tokenization, morphological analysis, named entity recognition, syntactic analysis, parsing, topic detection, and sentiment analysis.

Still in a further, either supplementary or alternative, embodiment, the natural language processing entity comprises or is at least functionally connected to a database such as a knowledgebase, or other entity, including a basic logic entity common to a plurality of natural languages supported by the arrangement and a language-specific language model and/or other language-specific definition(s) incorporating e.g. at least part of associated grammar, a vocabulary/dictionary, and/or a number of common phrases.

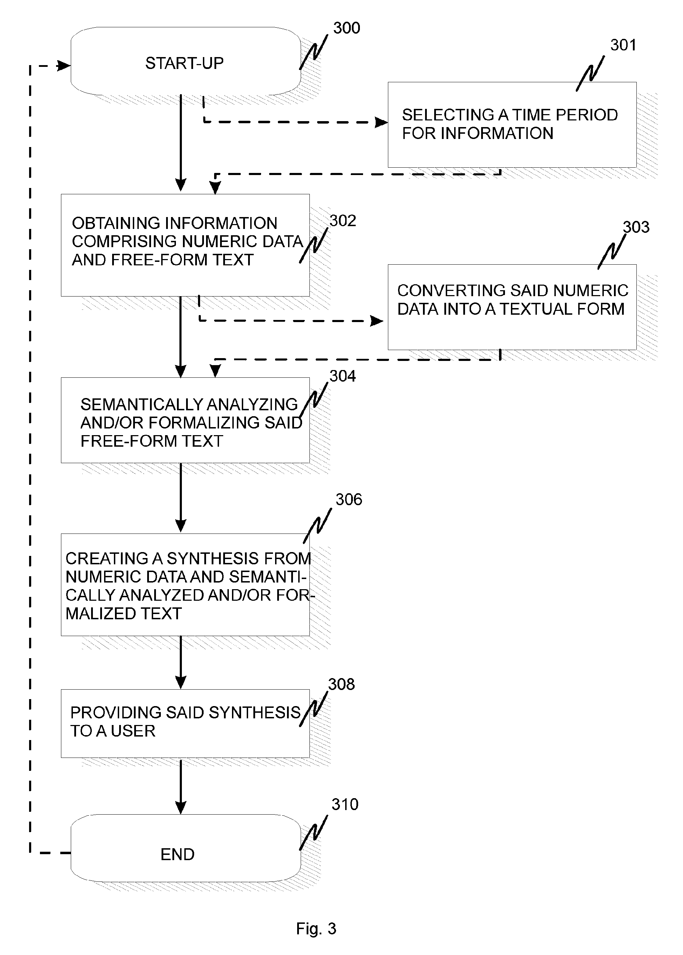

Fig. 3 discloses a merely exemplary flowchart in accordance of the present invention. The method may be executed by an embodiment of the arrangement disclosed herein.

At step 300, the method is started. This step may comprise required installation and/or initialization procedures, such as obtaining and configuring related elements and executing related software.

At step 302, the information comprising data in a numerical form and free-form text in natural language format is obtained. Depending on embodiment, the first information, the second information and, optionally, the third information and/or the fourth information are obtained. It is obvious to the skilled person that the obtained information is preferably saved in the memory entity of the control entity for later use. In one embodiment, the user can select what data is included in the synthesis, i.e. data from one or more devices in the group of external measurement can be included or excluded.

In one embodiment, the method comprises step 301 before step 302, wherein a time period for information is selected. In one embodiment, at step 302, only that information falling within the selected time period is obtained, and in another embodiment all available information is obtained, but the information falling within the selected time period is included in the material, from which the synthesis is created. It is also possible to execute step 301 after step 302, in which case the time period affects to the information, from which the synthesis is created. In one embodiment, the user is able to select different time period for different information, i.e. the first information can comprise the past two weeks and the second information the past month.

In another, either supplementary or alternative, embodiment, the method comprises a step of selecting and/or editing data weighing criteria. This step can be performed before or after step 301, but it is preferably performed after step 302.

At step 303, data in a numerical form is converted into a textual form. In one embodiment, the data in a numerical form comprises the first information from a wellbeing device and/or data in a numerical form provided by one or more external measurement device. The arrangement can comprise a knowledgebase of the correspondence between the numerical data and their textual form. Depending on embodiment, the numerical data provide by a wellbeing device can have its own textual correspondence and, respectively, each of the external measurement device its own. In one embodiment, the database of the correspondence is loaded in the memory entity of the arrangement or in some other way pointed, where the processor entity is able to find it. In another embodiment, a new device included in the arrangement is identified and, based on its data type, a suitable common knowledgebase is used to converting the numerical data into a textual form.

However, it is obvious to the person skilled in the art that some data may remain in a numerical form. It is advantageous to present well-known information, such as blood sugar values in a numerical form. Anyhow, an explanatory text can still be added to the value in question.

At step 304, the obtained free-form text in natural language format is semantically analyzed and/or formalized. The semantic analyzing and/or formalizing is performed by executing one or more various analyzing methods, such as, but not limited to, classification, filtering, language identification, segmentation, tokenization, morphological analysis, named entity recognition, syntactic analysis, parsing, topic detection, and sentiment analysis. The purpose of this semantic analyzing and/or formalizing is to provide formalized and comparable information in a textual form relating to the individual's wellbeing. The skilled person will understand that other methods can also be used to analyzing and/or formalizing the obtained free-form text in natural language format.

At step 306, the synthesis is created from the semantically analyzed and/or formalized text and the numerical data, which is converted into a textual form. In another embodiment, also other data, such as data in a numerical form, is included in the information for creating the synthesis. Depending on embodiment, the synthesis is created for the selected time period. In one embodiment, the synthesis is created by analyzing similarities between data of different information sources, combining the information with each other and removing repeating parts of the information. In another embodiment, the information is further categorized depending on its type and significance. Yet, in another embodiment, alterations during the selected time period are observed, and in another embodiment, a minor issue/issues occurred in more than one data source is disclosed.

At step 308, the synthesis is provided to a user. In one embodiment, the synthesis is provided in a textual form, such as an electronic text comprising one or more text files, which can be in different types of formats. In another embodiment, the synthesis can also comprise another than textual information, such as data in a graphical form and/or numerical data.

The synthesis can be presented in one or more electronic device, such as, but not limited to, a computer, a laptop, a tablet, a smartphone or another mobile device. The synthesis can also be converted into a speech form and read out loud via loudspeaker and/or headphones. It is obvious to the skilled person that the synthesis can also be transferred elsewhere, e.g. in an e-mailbox. In addition, the synthesis is printable in a paper by using a printer, which can be a part of the arrangement or connected to some other electronic device.

The scope of the invention is determined by the attached claims together with the equivalents thereof. The skilled persons will again appreciate the fact that the explicitly disclosed embodiments were constructed for illustrative purposes only, and the scope will cover further embodiments, embodiment combinations and equivalents that better suit each particular use case of the invention.

## Claims

1. An arrangement for creating a synthesis from numerical data and textual information relating to wellbeing of an individual comprising
- a first information in a numerical form,
- a second information comprising free-form text in natural language format, and
- a control entity for obtaining said first information and said second information, wherein said control entity is arranged to semantically analyze said free-form text in natural language format of said second information in order to create a synthesis from said first information and said second information.

2. An arrangement for creating a synthesis according to claim 1 further comprising one or more external measurement devices arranged to provide a third information, which third information is included in the obtained information in control entity for creating said synthesis.

3. An arrangement for creating a synthesis according to any preceding claim further comprising one or more external databases, from which a fourth information is obtained and included in the other obtained information in control entity for creating said synthesis.

4. An arrangement for creating a synthesis according to any preceding claim, wherein said synthesis is provided in a textual form.

5. An arrangement for creating a synthesis according to any preceding claim, wherein said first information is provided by a wellbeing device, which is arranged to monitor said individual's wellbeing.

6. An arrangement for creating a synthesis according to any preceding claim, wherein said second information is provided by caregiver personnel.

7. An arrangement for creating a synthesis according to any preceding claim, wherein a synthesis is created from information of a selectable a time period.

8. A method for creating a synthesis from numerical data and textual information by using an arrangement according to any of claim 1- 7 comprising at least following steps:
- obtaining information comprising data in a numerical form and free-form text in natural language format,
- converting said data in a numerical form into a textual form,
- semantically analyzing and/or formalizing said free-form text in natural language format,
- creating a synthesis from said data in a numerical form and said semantically analyzed and/or formalized free-form text in natural language format,
- providing said synthesis to a user.

9. A method for creating a synthesis according to any of claim 8 comprising a further step of selecting a time period for information.
